# EUROPEAN PATENT APPLICATION

(11) **EP 1 153 604 A1**
(43) Date of publication of application: **14.11.2001**
(21) Application number: 99903920.9
(22) Date of filing: 15.02.1999
(51) Int. Cl.: A61K 31/35, A61K 31/78

(54) **ADP-RIBOSYLATION INHIBITORS AND REMEDIES FOR ENDOTOXIC BACTERIAL ENTERIC INFECTION CONTAINING PROANTHOCYANIDIN AS THE ACTIVE INGREDIENT**

(71) Applicant: THE NIKKA WHISKY DISTILLING CO., LTD., Tokyo 107 (JP); Noda, Masatoshi, Yotsukaido-city, Chiba-prefecure 284-0045 (JP)
(72) Inventor: NODA, Masatoshi, Yotsukaido-city Chiba-pref. 284-0045 (JP); KANDA, Tomomasa, Kashiwa-city Chiba-pref. 277-0885 (JP); YANAGIDA, Akio, Tokyo 125-0042 (JP); HIEDA, Kazuo, Kashiwa-city Chiba-pref. 277-0074 (JP)
(74) Representative: Laget, Jean-Loup
(86) International application number: JP9900648
(87) International publication number: WO0047204

(57) **Abstract**

A composition for the treatment and/or prevention of enterotoxin type bacterial infectious disease comprising, as an active ingredient, a substance containing proanthocyanidin derived from a naturally occurring substance such as an apple or grape extract, which can inhibit toxins produced by enteric infection causative bacteria of enterotoxin type, e.g., pathogenic vibrios (Vibrio cholerae, Vibrio parahaemolyticus); and a composition for the treatment and/or prevention of diphtheria, etc. based on the ADP-ribosylation inhibiting activity of proanthocyanidin; as well as a food additive and foodstuff containing proanthocyanidin or the composition used for the treatment and prevention of these infectious diseases.

## Description

### FIELD OF THE INVENTION

The present invention relates to an adenosine-5'-diphosphate (ADP)-ribosylation inhibitor comprising proanthocyanidin as an active ingredient. More particularly, the invention relates to a composition for the treatment and/or prevention of enterotoxin type bacterial infectious disease as well as use of proanthocyanidin as a food additive and/or as a foodstuff.

### BACKGROUND OF THE INVENTION

The 1998 WHO data indicate that the death toll from bacterial infections reached 20 millions all over the world. In particular, the report says that the victims of cholera amount to several million individuals per year worldwide. Even now when medical technologies involving antibiotics, vaccines or the like have been greatly improved, the number of patients has not been reduced. Furthermore, because of increased personal exchange and physical distribution accompanied by remarkable progress of international transportation in these years, traveler's diarrhea caused by Vibrio cholerae, a dysentery bacillus, toxigenic Escherichia coli, salmonella, etc. tend to be increasing even in developed countries.

Cholera is an infectious disease with a very high rate of fatality, which is caused by Vibrio cholerae and characterized by copious watery diarrhea as a major syndrome. Cholera is found all over the world, especially in Asia and Africa. Cholera is caused by such a mechanism that Vibrio cholerae enters the body via the mouth, causes an infection in the mucous membranes lining the lumen of the small intestine and proliferates to produce cholera toxin. Cholera toxin has a structure composed of two protein subunits which are known as A and B subunits and are as seen with toxins from a dysentery bacillus, toxigenic E. coli, intestinal hemorrhagic E. coli, etc. The B subunit binds to ganglioside GM1 receptor sites on the epithelial cell of the gut lumen. It is known that the A subunit of cholera toxin having an ADP-ribosylase activity catalyzes the ADP-ribosylation that results in activation of the stimulatory GTP-binding protein of the adenylyl cyclase system, known as GS α, an increased cAMP level in the epithelial cell of the gut loop and a massive secretion of water and electrolytes, leading to watery diarrhea (Med. Immunol.; 21, 359-365 (1994); Masatoshi Noda, SHONI-NAIKA (Children Internal Medicine), 28 (9), 1186-1193 (1996)).

Cholera is currently treated by transfusion which is a symptomatic therapy. Under the actual situation, there is neither method nor medicament for the basic treatment of cholera has been found. As a result of extensive studies, Masatoshi Noda who is one of the present inventors already developed a medicament that inhibits toxins produced by bacteria such as Vibrio cholerae, etc. which cause enteric infections and makes the toxins harmless, see "MEDICAMENT FOR TREATING ENTEROTOXIN TYPE BACTERIAL INFECTIONS" (Japanese Patent Application No. 6-505177, Masatoshi Noda, Hajime Nishiya and Morimichi Yamaguchi) and "GALLIC ACID ESTER DERIVATIVES AND DRUGS CONTAINING THE SAME AS ACTIVE INGREDIENTS" (Japanese Patent Application Laid-Open No. 10-273495, Masamichi Ueno, Izumi Takai, Hiroshi Ohi, Masakazu Adachi and Masatoshi Noda). However, the former involves problems of production costs, etc. due to various limitations since herb medicines are used as major ingredients. The latter requires further investigations such as safety tests since the effective components are synthetic compounds. Thus, it will take at least several years until the latter is put into practical use. It is the state of the art that any medicament with high safety to human and derived from naturally occurring substances has not yet been provided at a low drug price for the treatment of enterotoxin type bacterial infectious disease. Therefore, it is strongly desired to provide a medicament that meets the foregoing requirements.

### DISCLOSURE OF THE INVENTION

An object of the invention is to provide an ADP-ribosylation inhibitor derived from a naturally occurring substance and/or a highly safe composition for the fundamental treatment and/or prevention of enterotoxin type bacterial infectious disease, which can inhibit the activity of toxins produced by enteric infection-causing bacteria via enterotoxin type bacteria such as pathogenic vibrios (Vibrio cholerae, Vibrio parahaemolyticus) and make these toxins harmless.

The present inventors made expansive investigations on naturally occurring substances, especially those derived from edible plants, that could inhibit ADP-ribosylation catalyzed in vivo by toxins such as cholera toxin and could suppress watery diarrhea but did not adversely affect human. As a result, a substance showing an extremely effective ADP-ribosylation inhibiting action and activity for treating enterotoxin type bacterial infectious disease has been found in the extracts of edible plants such as an apple extract, a grape extract, etc. The present invention has thus been accomplished.

### BRIEF DESCRIPTION OF THE DRAWING

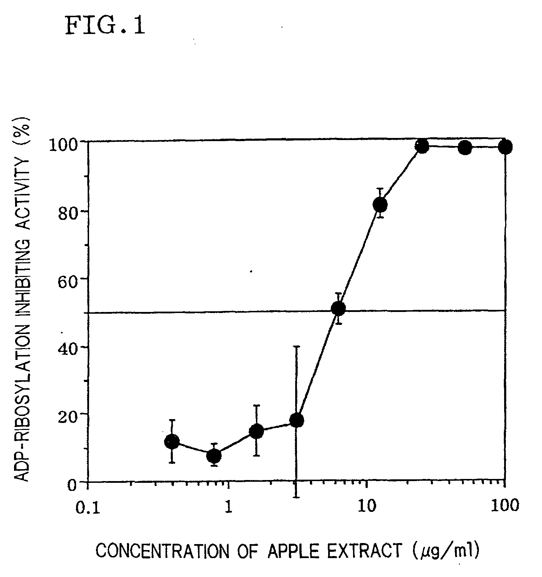

Fig. 1 is a graph showing the relationship between the concentration of an apple extract and its inhibitory activity in TEST EXAMPLE 1.

### BEST MODE FOR CARRYING OUT THE INVENTION

The apple extract which is one of the effective ingredients usable in the present invention consists essentially of polyphenols. Extensive studies on these polyphenols were already made by the present inventors with respect to compositional characteristics, safety and methods for preparation to confirm their functions and practical applicability, which was filed as Japanese Patent Application No. 7-285876. These polyphenols have been prepared by its applicant and marketed as food materials, food additives, cosmetic materials, etc. These commercial products have been actually applied to various foodstuffs, processed foods, cosmetics, etc. As a result of further extensive studies, the inventors have discovered in these polyphenols a substance showing an ADP-ribosylation inhibiting action effective for the treatment of enterotoxin type bacterial infectious disease; the inventors have identified this substance as proanthocyanidin which is one of the polyphenols.

Proanthocyanidin is a substance of at least dimers formed by condensation or polymerization of flavan-3-ol or flavan-3,4-diol having a flavonoidal skeleton (Hemingway, R. W. et al., Chemistry and Significance of Condensed Tannins, 83-107). It is confirmed that, for example, an apple extract contains 2- to 15-meric proanthocyanidin (Ohnishi-Kameyama M. et al., Rapid Comm. Mass Spectrom., 11, 31-36 (1997)).

Any extract can be used as the effective ingredient in the present invention, so long as the extract contains 2- to 15-meric proanthocyanidin derived from plants. Any plant material can be used irrespective of kind of plant, as far as the extract can be obtained from the plant material as containing a sufficient amount of the 2- to 15-meric proanthocyanidin. These plant extracts may of course be crude extracts but are required to contain at least a definite quantity of 2- to 15-meric proanthocyanidin. The extract may also be provided for use after fractionating into 2- to 15-meric proanthocyanidin and further purifying the fractions preferably to 4-to 15-meric proanthocyanidin, more preferably to proanthocyanidin of at least hexamer (6-mer). The fractionation and extraction of these proanthocyanidins of 2- to 15-mer, preferably 4- to 15-mer, more preferably at least 6-mer can be performed according to the method described in Japanese Patent Application No. 10-184143, which is herein incorporated by reference in its entirety.

Examples of edible plants containing proanthocyanidin include, in Rosaceae, strawberries and raspberries in addition to apples. Examples of edible plants other than those in Rosaceae are grapes in Vitaceae, kiwi fruits in Actinidia, blueberries and cranberries in Ericaceae, adzuki beans and tamarinds in Leguminosae, persimmons in Ebenaceae, bananas in Musaceae, cashew nuts in Anacardiaceae, cacaos in Sterculiaceae, Japanese oaks, evergreen oaks, chestnuts and pasanias in Fagaceae and, hops in Cannabinaceae. Among those plants, young apples removed as being superfluous, seeds contained in the residue of pressed grapes, etc. are advantageously used, in view of effective recycling of resources and a large extraction volume.

That is, the present invention provides an ADP-ribosylation inhibitor comprising as an effective ingredient proanthocyanidin derived from edible plants and a composition comprising the same for the treatment of enterotoxin type bacterial infectious disease. In addition to proanthocyanidin, the inhibitor and composition of the present invention may further contain other polyphenols, proanthocyanidin to which organic acids or sugars are bound, and solvates thereof such as hydrates. Other components obtained from the same raw material may also be contained in the inhibitor and composition of the present invention.

The pharmaceutical composition of the invention which comprises as an effective ingredient proanthocyanidin derived from edible plants is useful for the treatment and/or prevention of enterotoxin type bacterial infectious disease such as traveler's diarrhea caused by cholera or botulinus. Furthermore, since the pharmaceutical composition of the invention provides an excellent effect as an ADP-ribosylation inhibitor, the composition is also effective for the treatment and/or prevention of other infectious disease such as diphtheria, pertussis, tetanus, opportunistic infection, etc., the outbreaks of which are known to be closely associated with ADP-ribosylase.

The pharmaceutical composition of the invention comprising as an effective ingredient proanthocyanidin derived from edible plants can be prepared using carriers, excipients and other additives conventionally used to make pharmaceutical preparations. Either solid or liquid carriers and excipients may be used for making pharmaceutical preparations. Examples of such carriers and excipients include lactose, sucrose, fatty acid esters, dextrin, magnesium stearate, starch, talc, gelatin, agar, pectin, gum arabic, olive oil, sesame oil, cacao butter, ethylene glycol, etc.

The pharmaceutical composition of the invention is orally administered generally in the form of tablets, pills, capsules, granules, powders, liquid, etc. Oral dose may be appropriately determined on a case-by-case basis, depending upon conditions, age, sex, etc. of patients but the composition of the invention may be administered to adult generally in a daily dose of 100 mg to 10 g, preferably 200 mg to 1 g, when calculated as proanthocyanidin. The composition may be administered in a bolus mode, or the daily dose is divided into several times for oral administration. Of course, the daily dose may vary depending upon various conditions and in some case it may be sufficient in a smaller dose than that given above.

For oral administration of the composition in the present invention, a solid composition is available in the form of, e.g., tablets, powders or granules. In such a solid composition, edible plant-derived proanthocyanidin is blended with at least one diluent inert to the active ingredient. Examples of the diluent include lactose, mannitol, glucose, hydroxypropyl cellulose, microcrystalline cellulose, starch, metasilicic acid and magnesium aluminate. The composition may further contain additives other than the inert diluent, if desired and necessary. Examples of such additives include a lubricant such as magnesium stearate, a disintegrating agent such as calcium cellulose glycolate, a stabilizer such as lactose, a dissolution aid such as glutamic acid or aspartic acid. Tablets or pills may be coated, if necessary and desired, with an enteric or gastric coating film of sucrose, gelatin, hydroxypropyl cellulose, hydroxypropylmethyl cellulose phthalate, reduced palatinose, zein, etc.

A liquid composition available for oral administration containing edible plant-derived proanthocyanidin as an effective ingredient may be provided in the form of a pharmaceutically acceptable emulsion, solution, suspension, syrup, elixir, etc. These liquid preparations for oral administration may also contain an inert diluent conventionally employed, e.g., purified water or ethanol. In addition to the inert diluent above, the liquid preparations may further contain a wetting agent, a suspending agent, a wetting agent, an emulsifier, a dispersing agent, a stabilizer (e.g., lactose), an auxiliary agent such as a dissolution aid (e.g., glutamic acid, aspartic acid), a sweetener, a corrigent, flavor and a preservative.

The edible plant-derived proanthocyanidin of the invention can also be used as a food additive. In this case, prophylactic effects can be anticipated. Where the foodstuffs to be applied are liquid such as milk, beverages, juice, etc., the proanthocyanidin may be added to these liquid foodstuffs immediately before drinking. The amount of the proanthocyanidin used ranges from approximately 50 mg to 1,000 mg/100 ml of the liquid. Where the proanthocyanidin is incorporated into the staple food such as pasta, bread or rice, the proanthocyanidin can be added to the staple food in an amount of approximately 50 mg to 500mg/100 g of the hood, when the staple is cooked or processed.

### EXAMPLES

Hereinafter the present invention will be described in more detail with reference to the following EXAMPLES but is not deemed to be limited thereto.

### PREPARATION EXAMPLE 1 Preparation of apple extract

After 10 kg of young apple fruits were washed, crushed and then squeezed, the resulting juice was centrifuged and filtered to give 8.5 kg of clear juice. The juice was passed through a styrene-made adsorption resin column of 1 liter volume. After washing the column with water, elution was conducted with 65% aqueous ethanol solution. The eluate thus obtained was condensed in vacuo. The condensate was spray dried to give 59.7 g of the apple extract.

### PREPARATION EXAMPLE 2 Preparation of grape extract

After removing stalks, grapes were pressed and the residue of the pressed grapes was sieved to collect seeds. After 1 kg of the seeds were washed with water, the seeds were extracted with 50% ethanol. The extract was condensed in vacuo. The condensate was filtered to give the extract. The extract was then passed through a styrene-made adsorption resin column of 1 liter volume. After washing the column with water, elution was conducted with 65% aqueous ethanol solution. The eluate thus obtained was concentrated in vacuo and then spray dried to give 40.7 g of the grape extract.

### PREPARATION EXAMPLE 3 Fractionation of the apple extract

An aqueous solution of the apple extract obtained in PREPARATION EXAMPLE 1 was passed through a styrene-made adsorption resin column to give the total proanthocyanidin fraction containing 89% proanthocyanidin. The total proanthocyanidin fraction was applied to high performance liquid chromatography to fractionate into the monomer (catechins), dimer, trimer, tetramer and pentamer fractions and the fraction of hexamer or larger, respectively. Each of these fractions was concentrated in vacuo and lyophilized to give the dry product, respectively.

### PHARMACEUTICAL PREPARATION 1

The apple extract, 50 g, obtained in PREPARATION EXAMPLE 1 was mixed with 30 g of corn starch, 10 g of crystalline cellulose, 4 g of calcium carboxymethyl cellulose and 1 g of magnesium stearate. The mixture was tableted with a tableting machine to give tablets.

### PHARMACEUTICAL PREPARATION 2

The apple extract, 50 g, obtained in PREPARATION EXAMPLE 1 was mixed with 7 g of calcium carboxymethyl cellulose, 2 g of magnesium stearate and 1 g of silicic anhydride. A sufficient quantity of sterile water was added to the mixture. The mixture was extruded for granulation and dried to give granules.

### PHARMACEUTICAL PREPARATION 3

The apple extract, 45 g, obtained in PREPARATION EXAMPLE 1 was mixed with 5 g of sucrose fatty acid ester. The mixture was tableted with a tableting machine to give tablets. The tablets were coated with 25 g of reduced palatinose and further with 2 g of zein to give dragees.

### TEST EXAMPLE 1 Assessment by the agmatine assay

It is known that cholera toxin ADP-ribosylates Gs α,which is intracellular protein G, and also has a ADP-ribosyltransferase activity from NAD to ADP-ribose as a substrate for agmatine. Based on this mechanism, the activity of proanthocyanidin for inhibiting cholera toxin was assessed according to the agmatine assay reported by Noda et al. (Noda M. et al., Biochemistry, 28, 7936 (1989)).

That is, 1*µ*g of cholera toxin and a given amount of sample shown in Table 1 were mixed with the reaction mixture of 50 mM potassium phosphate buffer (pH 7.5), 5 mM MgCl₂, 100 *µ*M GTP, 100 *µ*M [adenine-¹⁴C] NAD (6000 cpm), 20 mM DTT, 20 mM agmatine and 0.1 mg/ml of ovalbumin (300 *µ*1 in total). The mixture was reacted at 30°C for 3 hours. The reaction mixture, 50 *µ*l, was passed through a column (φ5 x 20 mm) packed with Dowex AG1-X2 (manufactured by Bio Rad, Inc.) . After unreacted [adenine-¹⁴C] NAD was removed, the radioactivity of [adenine-¹⁴C] ADP-ribosylagmatine produced by ADP-ribosylation of cholera toxin was assayed. Using as an index this [adenine-¹⁴C] ADP-ribosylagmatine formation, the inhibition of the ADP-ribosyltransferase activity of cholera toxin by each sample was determined. The results are shown in Table 1. A graph showing the relationship between the concentration of the apple extract and the ADP-ribosylation inhibiting activity is also given in Fig. 1.

**Table 1**

| Cholera toxin inhibition rate (%) | | |
|---|---|---|
| Sample tested | Concentration (*µ*g/ml) | Inhibition rate (%) |
| Apple extract (young fruit)*¹ | 25 | 95.4 |
| Grape extract (seeds) | 63 | 96.1 |
| Sunflower extract (seeds) | 167 | 4.0 |
| Rhubarb extract (herb medicine)*² | 10 | 99.3 |
| Total proanthocyanidin fraction | 25 | 97.8 |
| monomer (catechins) | 25 | 0.0 |
| dimer fraction | 25 | 7.0 |
| trimer fraction | 25 | 9.5 |
| tetramer fraction | 25 | 51.8 |
| pentamer fraction | 25 | 96.7 |
| fraction of ≦ hexamer | 25 | 97.6 |

| | | |
|---|---|---|
| *1: apple extract IC 50 < 10 *µ*g/ml | | |
| *2: patients with positive response to cholera toxin: Japanese Patent Application No. 6-505177 | | |

In this test, the apple extract and the grape extract contained 51% and 63% of proanthocyanidin, respectively, when calculated as proanthocyanidin B2 according to the Porter method.

### TEST EXAMPLE 2 Assay by the ligated rabbit intestinal loop method

It is known that cholera toxin ADP-ribosylates Gs α known as intracellular protein G that results in persistent activation of adenylyl cyclase, an increased cAMP level in the epithelial cell of the gut loop and a massive secretion of water and electrolytes, leading to watery diarrhea. In order to determine the activity of inhibiting diarrhea caused by cholera toxin, the fluid accumulation activity of the apple extract in watery diarrhea was examined by the ligated rabbit loop method reported by De et al. (De S.N. and D.N. Chatterje, J. Pathol. Bacteriol., 66, 559 (1953)). That is, the rabbit ileum was ligated at every 15 cm to form several loops of the closed gut system. After 1 *µ*g of cholera toxin and a specified dose of the sample were given to each loop, the gut loop was returned into the body of the rabbit. The rabbit was kept as comfortable as possible and the fluid accumulation caused by cholera toxin was examined 18 hours later.

The results reveal that the apple extract suppressed almost completely the fluid accumulation caused by 1µg of cholera toxin at a dose of 50 *µ*g/loop. It was verified from these results that the edible plant extract described in the present invention and its effective ingredient proanthocyanidin act on cholera toxin to inhibit the ADP-ribosyltransferase activity and thus exhibit anti-cholera toxin activity, as does the extract of herb medicine "rhubarb".

### INDUSTRIAL APPLICABILITY

The ADP-ribosylation inhibitor of the invention comprising proanthocyanidin as an active ingredient is effective for the treatment and/or prevention of enterotoxin type bacterial infectious disease. The safety of proanthocyanidin as the active ingredient which is contained in such edible plant extracts as described above has already been established, since plants from which proanthocyanidin is derived have been utilized as edible plants in the long history of man. Even so, the safety of proanthocyanidin has been confirmed again prior to its use as a food additive by acute toxicity tests, etc. Proanthocyanidin has already been used widely as an extract from edible plants, an antioxidant or a food material. Any adverse effect to human is not reported at all. Thus, proanthocyanidin is extremely safe, very effective for the treatment and/or prevention of enterotoxin type bacterial infectious disease. Therefore, proanthocyanidin can provide an extremely useful medicament.

## Claims

1. An adenosine-5'-diphosphate (ADP)-ribosylation inhibitor comprising proanthocyanidin as an effective ingredient.

2. An ADP-ribosylation inhibitor according to claim 1, wherein proanthocyanidin is obtained from an edible plant or an edible plant-derived material.

3. An ADP-ribosylation inhibitor according to claim 2, wherein said edible plant or edible plant-derived material is an extract from an apple or a grape.

4. An ADP-ribosylation inhibitor according to any one of claims 1 through 3, wherein proanthocyanidin is the one extracted with at least one solvent selected from water, an alcohol, an ester and a ketone.

5. An ADP-ribosylation inhibitor according to any one of claims 1 through 4, wherein proanthocyanidin is the one purified using a styrene type adsorption resin, an anionic exchange resin, an octadecyl-chemically binding type silica gel, an octyl-chemically binding type silica gel, a phenyl-chemically binding type silica gel and a silica gel.

6. A composition for the treatment and/or prevention of diphtheria, pertussis, tetanus and opportunistic infection, comprising as an effective ingredient an ADP-ribosylation inhibitor according to any one of claims 1 through 5.

7. A composition for the treatment and/or prevention of enterotoxin type bacterial infectious disease comprising proanthocyanidin as an effective ingredient.

8. A composition for the treatment and/or prevention of enterotoxin type bacterial infectious disease according to claim 7, which is for the treatment and/or prevention of cholera, botulinus and traveler's diarrhea.

9. A composition for the treatment and/or prevention of enterotoxin type bacterial infectious disease according to claim 7 or 8, wherein said edible plant or edible plant-derived material is an extract from an apple or a grape.

10. A composition for the treatment and/or prevention of enterotoxin type bacterial infectious disease according to any one of claims 7 through 9, wherein proanthocyanidin is the one extracted with at least one solvent selected from water, an alcohol, an ester and a ketone.

11. A composition for the treatment and/or prevention of enterotoxin type bacterial infectious disease according to any one of claims 7 through 10, wherein proanthocyanidin is the one purified using a styrene type adsorption resin, an anionic exchange resin, an octadecyl-chemically binding type silica gel, an octyl-chemically binding type silica gel, a phenyl-chemical type binding silica gel and a silica gel.
